# EUROPEAN PATENT APPLICATION

(11) **EP 4 714 965 A1**
(43) Date of publication of application: **25.03.2026**
(21) Application number: 24825194.4
(22) Date of filing: 14.06.2024
(51) Int. Cl.: C07K 14/155, C07K 19/00, C12N 15/62, G01N 33/68, G01N 33/569

(54) **RECOMBINANT ANTIGEN FOR DETECTING HIV ANTIBODY**

(30) Priority: 21.06.2023 CN 202310748735
(71) Applicant: Fapon Biotech Inc., Shenzhen, Guangdong 518000 (CN)
(72) Inventor: LI, Ruijing, Shenzhen, Guangdong 518000 (CN); WEN, Piao, Shenzhen, Guangdong 518000 (CN); QIN, Tang, Shenzhen, Guangdong 518000 (CN); HONG, Haolin, Shenzhen, Guangdong 518000 (CN); CHENG, Qian, Shenzhen, Guangdong 518000 (CN); DAI, Shuang, Shenzhen, Guangdong 518000 (CN)
(74) Representative: Müller Schupfner & Partner Patent- und Rechtsanwaltspartnerschaft mbB (Hamburg)
(86) International application number: PCT/CN2024/099262
(87) International publication number: WO 2024/260298

(57) **Abstract**

Provided is an HIV recombinant antigen for detecting an HIV antibody, which belongs to the technical field of immunodiagnosis. The present application specifically relates to an HIV recombinant antigen, a nucleic acid molecule, an expression vector, a host cell, a reagent, a kit, and uses thereof. In the present invention, the recombinant HIV antigen protein is used in a label end, which can significantly improve the marker activity of the recombinant HIV antigen, reduce non-specific immune binding activity, and enhance sensitivity.

## Description

### Cross-Reference to Related Application

The present application claims the priority to the Chinese patent application No. 2023107487351, titled "RECOMBINANT ANTIGEN FOR DETECTING HIV ANTIBODY", filed to the China National Intellectual Property Administration on June 21, 2023, which incorporated herein in its entirety by reference.

### Technical Field

The present invention belongs to the field of immunodiagnostic technology and relates to a recombinant antigen for detecting an HIV antibody.

### Background

The following description provides only background information related to the present invention and does not necessarily constitute the prior art.

Currently, there are two types of HIV (human immunodeficiency virus, i.e., AIDS virus), namely HIV-1 and HIV-2. HIV-1 is currently the main virus causing AIDS. According to different cross-species transmission chains, HIV-1 can be classified into subtypes M, N, O, and P. Among them, subtype M accounts for 90% of HIV-1 infections. According to different epidemic regions, subtype M can be further divided into subtype A, B, C, D, E, F, G, H, I, J, K, and L. HIV-2 exhibits weaker transmission capability and lower strain virulence compared with HIV-1. Currently, there are eight known types of HIV-2, and only two types cause large-scale epidemics, namely group A and group B.

HIV is a spherical particle with a diameter of 100-120 nm, consisting of two parts: a core and an envelope. Its inner region is composed of a conical core enclosed by a structural protein P24 (capsid). The core consists of an ssRNA genome (enclosed by a p7 nucleocapsid), a reverse transcriptase, an integrase, a protease, several minor proteins, and major core proteins. A limited number of HIV envelope glycoproteins (Env) can be found on the surface of a virion, which are responsible for binding to its main host receptor CD4 and co-receptors (mainly CCR5 or CXCR4), leading to the entry of the virus into its target cells. Among them, the env gene of HIV-2 encodes a gp160 precursor protein, which is cleaved into a gp125 protein and a gp36 protein by the host cell protease furin in the host somatic cells.

HIV antibody detection is one of the main methods for screening HIV-infected patients. The gp36 protein is the main antigen raw material for HIV-2 antibody detection. However, the HIV gp36 has extremely strong hydrophobicity, which usually makes it difficult to prepare products with good detection performance.

### Summary

A main purpose of the present invention is to provide a gp36 protein sequence as shown in SEQ ID NO: 1 for detecting an HIV-2 antibody.

In some embodiments of the present invention, a portion of the gp36 protein sequence shown in SEQ ID NO: 1 is used to detect the HIV-2 antibody, such as a segment of amino acids from position x to position y of the gp36 protein, wherein x is any integer from 1 to 60, and y is any integer from 143 to 161.

Specifically, x is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60; and y is 143, 144, 145, 146, 147, 148, 149, 150, 151, 152, 153, 154, 155, 156, 157, 158, 159, 160, or 161.

In some embodiments of the present invention, the HIV recombinant antigen includes a segment of amino acids 1-143 of gp36 as shown in SEQ ID NO: 2, a segment of amino acids 15-143 as shown in SEQ ID NO: 3, or a segment of amino acids 60-161 as shown in SEQ ID NO: 4.

In some embodiments of the present invention, the cysteine in the recombinant antigen is mutated to other amino acids. In optional embodiments, the cysteine is mutated to serine.

In some embodiments of the present invention, the recombinant antigen further includes segments of other HIV proteins. In an optional embodiment, the recombinant antigen further includes a segment of HIV-1. In an optional embodiment, the recombinant antigen further includes a segment of HIV-1 gp41.

In some embodiments of the present invention, the recombinant antigen further includes a fusion partner.

Another aspect of the present invention provides a reagent/kit for detecting HIV, including the aforementioned recombinant antigen.

Specifically, the recombinant antigen in the reagent/kit is conjugated with a tracer marker or a solid-phase carrier.

Yet another aspect of the present invention provides a nucleic acid molecule encoding the aforementioned HIV recombinant antigen, a vector including the nucleic acid molecule, and a host cell including the vector.

### Brief Description of the Drawings

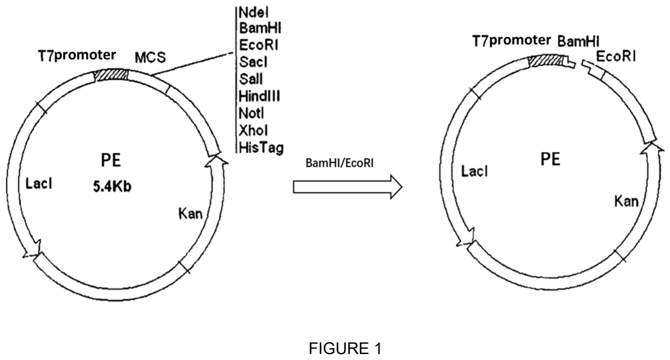

FIGURE 1 shows a map of an expression vector: the PE vector itself contains a 6×His tag at the N-terminus (other purification tags may also be used), wherein the translated HIV protein/recombinant protein includes a 6-histidine tag which is able to promote NI-NTA-assisted affinity purification.

### Detailed Description of the Embodiments

The use of the word "a" or "an" when used in conjunction with the term "comprising" in the claims and/or the specification may mean "one", but it is also consistent with the meaning of "one or more", "at least one", and "one or more than one".

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

Throughout this application, the term "about" is used to indicate that a value includes the standard deviation of error for the device or method being employed to determine the value.

The use of the term "or" in the claims is used to mean "and/or" unless explicitly indicated to refer to alternatives only or the alternatives are mutually exclusive, although the invention supports the definition of the term "or" that refers to only alternatives and "and/or."

When detecting antibodies in a sample with an antigen reagent, the antigen fragment acts as the reagent typically undergoes certain technical treatments. Therefore, the gp36 antigen described in the present invention may be either a full-length gp36 protein or a truncated gp36 protein. By way of example, the full-length gp36 protein includes the amino acids as shown in SEQ ID NO: 1, or it may be a truncated gp36 protein based on the full-length one, including the amino acids from position X to position Y of the gp36 protein, where X is any integer selected from 1 to 60, and Y is any integer selected from 143 to 161. By way of non-limiting examples, the truncated gp36 protein includes, for instance, the amino acids at positions 1-158, 1-153, 1-148, 1-143, 2-161, 5-158, 8-155, 11-152, 14-148, 15-143, 15-145, 15-147, 25-161, 135-161, 45-161, 50-161, 55-161, and 60-161 of the gp36 protein. For example, the gp36 antigen includes a sequence that has at least 90%, at least 91%, at least 92%, at least 93%, at least 94%, at least 95%, at least 96%, at least 97%, at least 98%, or at least 99% identity to SEQ ID NO: 1, SEQ ID NO: 2, SEQ ID NO: 3, or SEQ ID NO: 4.

As used herein, the term "antibody" in the present invention is used in the broadest sense, and may include a protein that includes an antigen-binding site. This encompasses natural antibodies and artificial antibodies of diverse structures, including but not limited to monoclonal antibodies, polyclonal antibodies, multi-specific antibodies (e.g., bispecific antibodies), single-chain antibodies, intact antibodies, and antibody fragments.

The term "fusion partner" is sometimes also referred to as a tag polypeptide, which is fused or connected to a target protein (e.g., the HIV recombinant antigen of the present invention) and thereby facilitates soluble expression, stabilization, and/or purification of the recombinant protein. The tag may be fused or linked to the N-terminus and/or C-terminus of the target protein (optionally via a linker or protease cleavage site). Such tags are well-known to those skilled in the art and have been described in detail in existing technical literature. For example, such tags include but are not limited to histidine (His) tags, glutathione S-transferase (GST) tags, maltose-binding protein (MBP) tags, thioredoxin (Trx) tags, NusA tags, disulfide isomerase DsbA tags, DsbC tags, SUMO tags, msyB tags, TF tags, trigger factor tags, ubiquitin tags, Myc tags, Flag tags, fluorescent protein (e.g., GFP) tags, biotin tags, avidin tags, and Foldon domains.

The term "peptide linker" or "linker peptide" refers to a short peptide used to connect two molecules (e.g., proteins). The HIV chimeric antigen of the present invention may include a linker peptide. Generally, a fusion protein (e.g., target protein 1-linker peptide-target protein 2, more specifically such as gp36 antigen-linker peptide-fusion partner, or gp36 antigen-linker peptide-other HIV fragments) is obtained by introducing (e.g., via PCR amplification or ligase) a polynucleotide sequence encoding the short peptide between two DNA fragments respectively encoding the two target proteins to be connected, followed by protein expression.

As used in the present invention, the term "recombinant protein" refers to a protein obtained using recombinant DNA or recombinant RNA technology, which can be obtained either in vivo or in vitro.

As used in the present invention, the term "polypeptide" refers to any molecule which may include three or more amino acid residues joined by peptide bonds. The polypeptide according to the present application includes peptides (e.g., tripeptides, oligopeptides, etc.), and may include chemically modified peptides (e.g., glycosylated peptides (glycopolypeptides), phosphorylated peptides, hydroxylated peptides, sulfonated peptides, palmitoylated peptides, and disulfide bond-formed peptides). The polypeptide may also refer to a protein.

As used in the present invention, the term "signal peptide" refers to a polypeptide connected to a target protein that is able to promote expression or transfer of the target protein. By way of example, the signal peptide may be connected to the N-terminus of the target protein and is generally cleavable for removal, wherein it is not present in the mature protein secreted by the cells.

As used in the present invention, the term "amino acid mutation" or "nucleotide mutation" includes "substitution, repetition, deletion or addition of one or more amino acids or nucleotides". In the present invention, the term "mutation" refers to a change in a nucleotide sequence or an amino acid sequence. In some embodiments, the "mutation" of the present invention may be selected from "conservative mutation", "semi-conservative mutation", and "non-conservative mutation". In the present invention, the term "non-conservative mutation" or "semi-conservative mutation" may be a mutation causing loss or partial loss of the protein function. The term "conservative mutation" refers to a mutation that may normally maintain the function of a protein. A representative example of the conservative mutation is a conservative substitution.

As used in the present invention, "conservative substitution" usually means an exchange of one kind of amino acid at one or more sites of a protein. Such a substitution may be conservative. Specifically, examples of the substitution taken as the conservative substitution may include a substitution of Ala with Ser or Thr, a substitution of Arg with Gln, His or Lys, a substitution of Asn with Glu, Gln, Lys, His or Asp, a substitution of Asp with Asn, Glu or Gln, a substitution of Cys with Ser or Ala, a substitution of Gln with Asn, Glu, Lys, His, Asp or Arg, a substitution of Glu with Gly, Asn, Gln, Lys or Asp, a substitution of Gly with Pro, a substitution of His with Asn, Lys, Gln, Arg or Tyr, a substitution of Ile with Leu, Met, Val or Phe, a substitution of Leu with Ile, Met, Val or Phe, a substitution of Lys with Asn, Glu, Gln, His or Arg, a substitution of Met with Ile, Leu, Val or Phe, a substitution of Phe with Trp, Tyr, Met, Ile or Leu, a substitution of Ser with Thr or Ala, a substitution of Thr with Ser or Ala, a substitution of Trp with Phe or Tyr, a substitution of Tyr with His, Phe or Trp, and a substitution of Val with Met, Ile or Leu. In addition, conservative mutations further include naturally occurring mutations caused by the difference in individuals from which the genes are derived and the differences in strains and species, etc.

In the present invention, "sequence identity" and "percent identity" refer to the percentage of the same (i.e., identical) nucleotides or amino acids between two or more polynucleotides or polypeptides. The sequence identity between two or more polynucleotides or polypeptides can be determined by the following method: aligning the nucleotide or amino acid sequences of the polynucleotides or polypeptides and scoring the number of positions at which nucleotide or amino acid residues are identical in the aligned polynucleotides or polypeptides, and comparing the number of these positions with the number of positions at which nucleotide or amino acid residues are different in the aligned polynucleotides or polypeptides. Polynucleotides may differ at one position, for example, by containing different nucleotides (i.e., substitutions or mutations) or deleted nucleotides (i.e., nucleotide insertions or nucleotide deletions in one or two polynucleotides). Polypeptides may differ at one position, for example, by containing different amino acids (i.e., substitutions or mutations) or deleted amino acids (i.e., amino acid insertions or amino acid deletions in one or two polypeptides). The sequence identity may be calculated by dividing the number of positions at which nucleotide or amino acid residues are identical by the total number of nucleotide or amino acid residues in the polynucleotides or polypeptides. For example, the percent identity may be calculated by dividing the number of positions at which nucleotide or amino acid residues are identical by the total number of nucleotide or amino acid residues in the polynucleotides or polypeptides, and multiplying the result by 100.

Examplary, in the present invention, two or more sequences or subsequences, when compared and aligned at maximum correspondence by the sequence alignment algorithm or by the visual inspection measurement, have at least 40%, 50%, 60%, 70%, 80%, 85%, 90%, 91%, 92%, 93%, 94%, 95%, 96%, 97%, 98%, or 99% "sequence identity" or " identity percent " of nucleotide or amino acid residues. The "sequence identity" or "identity percent" may be determined /calculated on the basis of any suitable region of the sequence. For example, a region of at least about 50 residues, a region of at least about 100 residues, a region of at least about 200 residues, a region of at least about 400 residues, or a region of at least about 500 residues in length. In some embodiments, the sequences are substantially identical over the entire length of either or both of the compared biopolymers (i.e., nucleic acids or polypeptides).

As used in the present invention, the term "polynucleotide" refers to a polymer composed of nucleotides. The polynucleotide may be in the form of a separate fragment or may be a component of a larger nucleotide sequence structure, which is derived from a nucleotide sequence that has been separated at least once in terms of quantity or concentration, and is capable of being identified, manipulated, and recovered in sequence along with its component nucleotide sequences using standard molecular biology methods (e.g., using cloning vectors). When a nucleotide sequence is represented by a DNA sequence (i.e., A, T, G, C), this also includes an RNA sequence (i.e., A, U, G, C), where "U" replaces "T". In other words, "polynucleotide" refers to a polymer of nucleotides removed from other nucleotides (individual fragments or entire fragments), or may be a component or constituent of a larger nucleotide structure, such as an expression vector or polycistronic sequence. The polynucleotide includes DNA, RNA, and cDNA sequences. "Recombinant polynucleotide" and "recombinant nucleic acid molecule" belong to one type of "polynucleotide".

As used in the present invention, the term "recombinant nucleic acid molecule" refers to polynucleotides having sequences that are not linked together in nature. The recombinant polynucleotide may be included in a suitable vector, and the vector may be used to transform into a suitable host cell. Then the polynucleotide is expressed in a recombinant host cell to produce, for example, "recombinant polypeptide", "recombinant protein", or "fusion protein".

As used in the present invention, the term "vector" refers to a DNA construct that contains a DNA sequence operably linked to a suitable control sequence to express a gene of interest in a suitable host.

As used in the present invention, the term "recombinant expression vector" refers to a DNA structure used to express, for example, a polynucleotide encoding a desired polypeptide. The recombinant expression vector may include, for example, a transcription subunit including i) a set of genetic elements having regulatory effects on gene expression, such as promoters and enhancers; ii) a structural or coding sequence transcribed into mRNA and translated into a protein; and iii) appropriate transcription and translation initiation and termination sequences. The recombinant expression vector is constructed in any suitable manner. The nature of the vector is not important, and any vector can be used, including plasmids, viruses, phages, and transposons.

The term "host cell" in the present invention means any cell type that is easily transformed, transfected, transduced, etc., with a gene editing element, a nucleic acid construct, or a recombinant expression vector containing the present invention. The term "recombinant host cell" encompasses a host cell that is different from a parent cell after the introduction of a gene editing element, a nucleic acid construct, or a recombinant expression vector, and the recombinant host cell is specifically achieved by transformation. The host cell of the present invention may be a prokaryotic cell or a eukaryotic cell, as long as it is a cell capable of introducing the recombinant nucleic acid molecule or recombinant expression vector of the present invention.

The terms "transformation, transfection, transduction" in the present invention have the meaning generally understood by those skilled in the art, that is, the process of introducing an exogenous DNA into a host. The methods of transformation, transfection, and transduction include any method of introducing nucleic acid into cells, including but not limited to electroporation, calcium phosphate (CaPO₄) precipitation, calcium chloride (CaCl₂) precipitation, and microinjection, polyethylene glycol (PEG) method, DEAE-dextran method, cationic liposome method, and lithium acetate-DMSO method.

As used in the present invention, the terms "sample", "specimen", "sample to be detected" or "test sample" refer to any type of sample for which it is necessary to determine whether HIV antibodies are present therein. By way of example, the sample to be detected may be any product produced by a subject, or any product derived from a product produced by a subject. The sample may be taken from any tissue or body fluid, such as a blood sample (including samples derived from blood), a serum sample, a lymph sample, a saliva sample, or synovial fluid. The sample derived from blood may be a selected portion of the blood from a patient or a vaccinated individual, such as a selected cell-containing portion, or a plasma or serum portion. In some embodiments, the sample may be any sample containing antibody products of the humoral immune response.

As used in the present invention, "diagnosis" includes the detection or identification of a disease state or condition of a subject, determining the likelihood that a subject will develop a given disease or condition, determining the likelihood that a subject with a disease or condition will respond to treatment, determining the prognosis (or its possible progression or regression) of a subject with a disease or condition, and determining the effect of a treatment on a subject with a disease or condition. For example, the diagnosis can be used for detecting the presence or likelihood of a subject being infected with HIV or the likelihood that such a subject will respond favorably to a compound (e.g., a pharmaceutical, e.g., a drug) or other treatment.

The terms "individual", "patient" or "subject" used in the context of the present invention include mammals. Mammals include, but are not limited to, domestic animals (e.g., cattle, sheep, cats, dogs, and horses), primates (e.g., humans and non-human primates such as monkeys), rabbits, and rodents (e.g., mice and rats).

The method disclosed in the present invention may be performed in vitro, ex vivo, or in vivo, or the product may exist in an in vitro, ex vivo, or in vivo form. The term "in vitro" refers to experiments that use materials, biological substances, cells, and/or tissues under laboratory conditions or in culture media; while the term "in vivo" refers to experiments and procedures that use intact multicellular organisms. In some embodiments, a method performed in vivo can be conducted on a non-human animal. "Ex vivo" refers to events that exist outside or occur outside an organism, such as events outside the human or animal body, for example, events that can exist or occur on tissues (e.g., entire organs) or cells taken from an organism.

### Technical Solution

In some optional embodiments, the HIV recombinant antigen includes a polypeptide having the amino acid sequence as shown in SEQ ID NO: 1, or a polypeptide derived from the amino acid sequence as shown in SEQ ID NO: 1 by substitution, repetition, deletion, or addition of one or more amino acids and having all or part of the biological activity of the sequence as shown in SEQ ID NO: 1.

In some optional embodiments, the HIV recombinant antigen includes a polypeptide having the amino acid sequence as shown in SEQ ID NO: 2, or a polypeptide derived from the amino acid sequence as shown in SEQ ID NO: 2 by substitution, repetition, deletion, or addition of one or more amino acids and having all or part of the biological activity of the sequence as shown in SEQ ID NO: 2.

In some optional embodiments, the HIV recombinant antigen includes a polypeptide having the amino acid sequence as shown in SEQ ID NO: 3, or a polypeptide derived from the amino acid sequence as shown in SEQ ID NO: 3 by substitution, repetition, deletion, or addition of one or more amino acids and having all or part of the biological activity of the sequence as shown in SEQ ID NO: 3.

In some optional embodiments, the HIV recombinant antigen includes a polypeptide having the amino acid sequence as shown in SEQ ID NO: 4, or a polypeptide derived from the amino acid sequence as shown in SEQ ID NO: 4 by substitution, repetition, deletion, or addition of one or more amino acids and having all or part of the biological activity of the sequence as shown in SEQ ID NO: 4.

In some optional embodiments, the HIV recombinant antigen includes a polypeptide having the amino acid sequence as shown in SEQ ID NO: 5, or a polypeptide derived from the amino acid sequence as shown in SEQ ID NO: 5 by substitution, repetition, deletion, or addition of one or more amino acids and having all or part of the biological activity of the sequence as shown in SEQ ID NO: 5.

In some optional embodiments, the HIV recombinant antigen includes a polypeptide having the amino acid sequence as shown in SEQ ID NO: 6, or a polypeptide derived from the amino acid sequence as shown in SEQ ID NO: 6 by substitution, repetition, deletion, or addition of one or more amino acids and having all or part of the biological activity of the sequence as shown in SEQ ID NO: 6.

In some optional embodiments, the HIV recombinant antigen includes a polypeptide having the amino acid sequence as shown in SEQ ID NO: 7, or a polypeptide derived from the amino acid sequence as shown in SEQ ID NO: 7 by substitution, repetition, deletion, or addition of one or more amino acids and having all or part of the biological activity of the sequence as shown in SEQ ID NO: 7.

In some optional embodiments, a linker peptide may be selected as a flexible linker peptide. The linker peptide includes but is not limited to GS, GGSGG (SEQ ID NO: 8), KESGSVSSEQLAQFRSLD (SEQ ID NO: 9), GSAGSAAGSGEF (SEQ ID NO: 10), GGSGGEFGGSGG (SEQ ID NO: 11), and variants thereof.

In some embodiments, the HIV recombinant antigen further includes a tag polypeptide at its N-terminus and/or C-terminus. By way of example, the tag polypeptide is a His tag, preferably a 6×His tag, which is linked to the N-terminus of the HIV antigen and allows purification by Ni-NTA affinity chromatography. In some optional embodiments, the HIV recombinant antigen and the tag polypeptide may be linked via a linker peptide.

In some embodiments, the HIV recombinant antigen is conjugated with a tracer marker or a solid-phase carrier; the tracer marker is selected from at least one of acridinium ester, luminol, isoluminol, alkaline phosphatase, horseradish peroxidase, colloidal gold, fluorescent microspheres, tris(bipyridine)ruthenium, quantum dot luminescent materials, and up-conversion luminescent materials; and the solid-phase carrier is selected from at least one of magnetic microspheres, plastic microspheres, plastic particles, microplates, glass, capillaries, nylon, and nitrocellulose membranes.

In some embodiments, the present invention provides a recombinant nucleic acid molecule including a nucleotide sequence encoding any of the aforementioned HIV recombinant antigens, and the recombinant nucleic acid molecule may be DNA, RNA, or a combination thereof.

In some embodiments, the present invention provides a recombinant expression vector, which includes the aforementioned recombinant nucleic acid molecule and can be used for transferring the recombinant nucleic acid molecule into cells. In some embodiments, the recombinant expression vector includes one or more regulatory elements, and the regulatory elements may be those commonly used in the art such as promoters, enhancers, silencers, and insulators. The regulatory elements are operably linked to the recombinant nucleic acid molecule to mediate the transcription and translation of the recombinant nucleic acid molecule.

The term "operably linked" may include a situation where a selected nucleic acid molecule sequence is covalently linked to a regulatory element sequence (e.g., a promoter and/or an enhancer) to place the expression of the nucleic acid sequence under the influence or control of the regulatory element (thereby forming an expression cassette). Therefore, if the regulatory element is capable of acting on the transcription of the nucleic acid molecule, the regulatory element is operably linked to the nucleic acid molecule. The obtained transcript can be translated into the desired polypeptide or protein.

In the present invention, vectors suitable for constructing recombinant expression vectors include plasmids, binary vectors, DNA vectors, mRNA vectors, viral vectors (e.g., gamma retroviruses (e.g., vectors derived from murine leukemia virus (MLV)), lentiviral vectors, adenoviral vectors, adeno-associated viral vectors, vaccinia viral vectors, and herpes viral vectors), transposon-based vectors, and artificial chromosomes (e.g., yeast artificial chromosomes) and variants thereof.

In some embodiments, the vector is a prokaryotic vector, which contains various elements expressed in prokaryotic cells. For example, the vectors include, but are not limited to, pET series vectors, Duet series vectors, pGEX series vectors, pHY300 vectors, pHY300PLK vectors, and PE vectors. Preferably, the vector is a PE vector.

In some embodiments, the present invention provides a recombinant host cell including the aforementioned recombinant nucleic acid molecule or recombinant expression vector. The recombinant host cell is obtained by transforming, transfecting, or transducing the recombinant nucleic acid molecule or recombinant expression vector into a host cell. The host cell in the present invention may be a eukaryotic cell or a prokaryotic cell, as long as it can introduce the recombinant nucleic acid molecule or recombinant expression vector of the present invention and achieve the expression of the recombinant receptor-binding protein.

In some embodiments, the host cell is a prokaryotic cell, such as a bacterial cell. The bacteria can be cocci (e.g., *Micrococcus, Thermococcus, Streptococcus*), bacilli (e.g., *Enterobacter, Bacillus, Actinobacillus, Acetobacter, Lactobacillus*), or spirilla (e.g., *Brachyspira*). In some embodiments, the host cell is *Escherichia coli* ER2529, *Escherichia coli* BL21 (DE3), etc.; and preferably *Escherichia coli* ER2566.

In some embodiments, the HIV recombinant antigen can be prepared by expression in a recombinant host cell, and the recombinant host cell may be a prokaryotic cell or a eukaryotic cell. In some embodiments, the recombinant host cell is a prokaryotic cell; for example, the recombinant host cell is obtained by introducing a recombinant expression vector into *Escherichia coli.*

The recombinant host cell is cultured under conditions suitable for protein expression. After the completion of culture, the recombinant HIV antigen protein is collected from the cell culture medium or fermentation broth, and then protein purification treatment is performed on the HIV recombinant antigen protein.

In some embodiments, the steps of performing protein purification treatment on the HIV recombinant antigen protein include Ni-NTA affinity chromatography, ion exchange chromatography, protein dialysis, and the like.

In some embodiments, provided is a method for diagnosing an HIV-related disease, including: contacting the aforementioned HIV recombinant antigen, reagent, or kit with an HIV antibody in a biological sample to detect the presence of the HIV-1 antibody in the biological sample.

In some optional embodiments, the aforementioned method includes performing an immunoassay on the biological sample, wherein the immunoassay includes ELISA, fluorescent immunochromatography, colloidal gold immunochromatography, chemiluminescence assay, electrochemiluminescence assay, indirect immunofluorescence assay (IFA), or radioimmunoassay (RIA).

Unless otherwise specified, the experimental techniques and methods used in the present embodiment are all conventional technical methods. For example, experimental methods without specified specific conditions in the following embodiments are generally performed in accordance with conventional conditions, such as those described in Sambrook et al., Molecular Cloning: A Laboratory Manual (New York: Cold Spring Harbor Laboratory Press, 1989), or in accordance with the conditions recommended by the manufacturer. Unless otherwise specified, the materials, reagents, and the like used in the embodiments can all be obtained through formal commercial channels.

### Example 1 Preparation of an HIV Recombinant Antigen

### 1.1 Preparation of gp36 Recombinant Antigen

### Plasmid Design:

According to the amino acid sequences shown in SEQ ID NO: 2, SEQ ID NO: 3 and SEQ ID NO: 4 respectively, a fusion partner GST was introduced at the N-terminus of a recombinant antigen, and an expression plasmid was constructed based on a nucleic acid encoding a protein sequence. The two ends of each gene were flanked by BamHI and EcoRI sites. After double digestion with BamHI and EcoRI, the mutated fragments were ligated into the pE vector (FIGURE 1) to generate the corresponding expression plasmids.

### Induced Expression:

The aforementioned constructed expression plasmid was transformed into *Escherichia coli* expression strain ER2566 (NEB) by a heat shock method, and plated on an LB plate containing 100 µg/ml Amp, followed by being cultured at 37°C for 16 h. Single colonies were picked, and positive strains identified as correct by colony PCR were selected for sequencing. After sequence verification, the strains were inoculated into an LB medium containing 50 µg/ml Amp and cultured with shaking at 37°C. When the OD600 reached 0.6-0.8, 1.0 mM IPTG was added for induction culture at 37°C for 2-4 h. The expression of the recombinant proteins was identified by SDS-PAGE. After purification, the obtained proteins were named HIV-G-1 (SEQ ID NO: 2), HIV-G-2 (SEQ ID NO: 3) and HIV-G-3 (SEQ ID NO: 4), respectively.

### 1.2 Preparation of gp36-gp41 Chimeric Antigen

A fusion antigen of gp36 (SEQ ID NO: 4) and gp41 (SEQ ID NO: 5) was prepared, that is, a gp41-gp36 chimeric sequence (SEQ ID NO: 5 + SEQ ID NO: 4, with a restriction enzyme cutting site amino acid residue RS between them) was designed. A recombinant protein HIV-G-4 (SEQ ID NO: 6), was obtained in the same manner as in Example 1.1.

### 1.3 Preparation of Cysteine Mutant Antigen

A cysteine-mutated antigen was designed based on the HIV-G-4 (wherein cysteine was mutated to serine). A recombinant protein HIV-G-5 (SEQ ID NO: 7) was obtained in the same manner as in Example 1.1.

### Example 2 Evaluation of Sensitivity and Specificity of the Recombinant HIV Antigen of the Present Invention by Double-Antigen Sandwich Method on a Colloidal Gold Chromatography Platform

### 2.1 Detection of HIV-2 Type Samples

### (1) HIV Antigen Labeling

5 mL of 4/10,000 colloidal gold was taken, an appropriate amount of 0.2 M K₂CO₃ was added and stirred for 5 min, an HIV-labeled antigen was added and stirred for 5 min, then an appropriate amount of 10% BSA was added to block and terminate the labeling; the mixture was centrifuged at 10,000 rpm for 10 min, the supernatant was removed, the precipitate was redissolved with a gold reconstitution solution, and finally, the volume was made up to 0.5 mL with the gold reconstitution solution (i.e., 1/10 of the colloidal gold solution volume); the labeled antigen-concentrated gold was dilute with the gold reconstitution solution to a certain multiple to prepare a gold working solution, and then the gold was spread; the spread gold was placed in a freeze dryer for freeze-drying (1-2 h) or dried overnight in a 37°C drying room;

### (2) HIV Antigen Coating

The HIV antigen was diluted to a concentration of 1.0 mg/ml with a coating dilution buffer (10 mM PB + 150 mM NaCl + 2% sucrose, pH 7.4) to coat the T-line with the HIV antigen, and placed in a 37°C constant-temperature incubator for 1-2 hours;

### (3) Preparation of Gold-Labeled Strips

Gold-labeled strips were cut into the required width using a strip cutter, the strips were assembled, and then samples were added for detection. It should be noted that the colloidal gold color card has 10 reading gradients in total, namely C1, C2, C3, C4, C5, C6, C7, C8, C9, and B, wherein the corresponding T-line color of these 10 gradients gradually lightened from left to right, C1 indicated the darkest T-line color, C9 indicated the lightest T-line color, B indicated no color development, and B+ indicated almost no color development; and in Table 2, "C3+" referred to a color slightly darker than C3 but not reaching C2, and the same rule was applied to other designations with a "+" sign.

### Test results:

The color development degree was interpreted using the color card, and the results are as Table 1.

**Table 1**

| Coated antigen | | HIV-2 antigen (Fapon Catalog No.: P82) | | |
|---|---|---|---|---|
| Labeled antigen | | HIV-G-1 | HIV-G-2 | HIV-G-3 |
| Sensitivity | Clinical positive sample 1 of HIV-2 | C1 | C2 | C1 |
| | Clinical positive sample 2 of HIV-2 | C2 | C2 | C2 |
| | Clinical positive sample 3 of HIV-2 | C2 | C2 | C1 |
| | Clinical positive sample 4 of HIV-2 | C3 | C3 | C3 |
| | Clinical negative sample 1 | B | B | B |
| | Clinical negative sample 2 | B | B | B |
| | Clinical negative sample 3 | B | B | B |
| | Clinical negative sample 4 | B | B | B |
| Specificity | Clinical negative sample 5 | B | B | B |
| | Clinical negative sample 6 | B | B | B |
| | Clinical negative sample 7 | B | B | B |
| | Clinical negative sample 8 | B | B | B |
| | Clinical negative sample 9 | B | B | B |
| | Clinical negative sample 10 | B | B | B |

The results demonstrated that the HIV-G-1, HIV-G-2, and HIV-G-3 of the present invention as antigens could all detect HIV-2 type samples with high sensitivity and specificity.

### 2.2 Detection of HIV Samples (Including Confirmed HIV-2 Positive Samples and Abbott HIV Positive Samples)

The operation procedure was the same as that in Example 2.1, except that two T-lines were coated on a test strip using a P81 antigen targeting HIV-1 and a P82 antigen targeting HIV-2, respectively.

### Test results:

The color development degree was interpreted using a color card, and the results were as Table 2 (the reading on the left side of the symbol "/" indicated the detection result of the HIV-2 antibody, while the reading on the right side indicated the detection result of the HIV-1 antibody).

**Table 2**

| Coated antigen | | HIV-2 antigen (Fapon Catalog No.: P81) + HIV-1 antigen (Fapon Catalog No.: P82) | |
|---|---|---|---|
| Labeled antigen | | HIV-G-4 | HIV-G-5 |
| | Clinical positive sample 1 of HIV-2 | C1/B | C1/B |
| | Clinical positive sample 2 of HIV-2 | C2/B | C2/B |
| | Clinical positive sample 3 of HIV-2 | C1/B | C2/B |
| | Clinical positive sample 4 of HIV-2 | C3+/B | C3+/B |
| | Clinical positive sample 1 | B/C2 | B/C2+ |
| | Clinical positive sample 2 | C9/C3 | C9/C3+ |
| | Clinical positive sample 3 | C9/C2 | C9/C2+ |
| | Clinical positive sample 4 | B/C2 | B/C2+ |
| | Clinical positive sample 5 | B/C3+ | B/C3+ |
| | Clinical positive sample 6 | B/C4 | B/C4 |
| Sensitivity | Clinical positive sample 7 | B/C4+ | B/C4+ |
| | Clinical positive sample 8 | B/C3 | B/C3 |
| | Clinical positive sample 9 | C9+/C4 | C9/C4 |
| | Clinical positive sample 10 | B/C3 | B/C3+ |
| | Clinical positive sample 11 | B/C3 | B/C3+ |
| | Clinical positive sample 12 | B/C2 | B/C2+ |
| | Clinical positive sample 13 | C9/C4 | C9/C4 |
| | Clinical positive sample 14 | C9/C2 | C9/C2+ |
| | Clinical positive sample 15 | B/C3+ | B/C2 |
| | Clinical positive sample 16 | C9/C3+ | C9/C2 |
| | Clinical positive sample 17 | C9/C4+ | C9/C4 |
| Specificity | Sample diluent | B/B | B/B |
| | Negative clinical serum | No positive results were observed in 90 tested samples. | No positive results were observed in 90 tested samples. |
| | Negative clinical plasma | No positive results were observed in 88 tested samples. | No positive results were observed in 88 tested samples. |

The results demonstrated that both HIV-G-4 and HIV-G-5 of the present invention as antigens could effectively detect HIV positive samples.

### Example 3 Investigation on Thermal Stability

The stability evaluation of the HIV recombinant antigen included an accelerated assessment at 37°C for 3 days and 7 days, and the formation of aggregates was detected by SDS-PAGE protein gel without mercaptoethanol treatment.

### 3.1 Aggregate Formation

By observing the SDS-PAGE protein gel of the HIV recombinant antigen stored at 4°C and evaluated under 37°C conditions for 3 days and 7 days without mercaptoethanol treatment, the aggregate formation was summarized in the following table, wherein less aggregate formation indicated better stability. It could be seen that the mutation of cysteine to serine in the HIV recombinant antigen could significantly improve its thermal stability (Table 4).

**Table 4**

| HIV recombinant antigen | Aggregate occurrence | | |
|---|---|---|---|
| | 37°C for 3 consecutive days | 37°C for 7 consecutive days | 4°C for 7 consecutive days |
| HIV-G-4 | Slight | Slight | None |
| HIV-G-5 | None | None | None |

### Related Sequences

SEQ ID NO: 1 
LATAGSAMGAASLTLSAQSRTLLAGIVQQQQLLDVVKRQQEMLRLTVWGTKNLQA
RVTAIEKYLKDQAQLNSWGCAFRQVCHTTVPWVNDSLSPDWKNMTWQEWEKQVRYLE
ANISQQLEQAQIQQEKNMYELQKLNSWDVFSNWFDLTSWIKYIQYGVY
SEQ ID NO: 2 
LATAGSAMGAASLTLSAQSRTLLAGIVQQQQLLDVVKRQQEMLRLTVWGTKNLQA
RVTAIEKYLKDQAQLNSWGCAFRQVCHTTVPWVNDSLSPDWKNMTWQEWEKQVRYLE
ANISQQLEQAQIQQEKNMYELQKLNSWDVF
SEQ ID NO: 3 
LSAQSRTLLAGIVQQQQLLDVVKRQQEMLRLTVWGTKNLQARVTAIEKYLKDQAQ
LNSWGCAFRQVCHTTVPWVNDSLSPDWKNMTWQEWEKQVRYLEANISQQLEQAQIQQ
EKNMYELQKLNSWDVF
SEQ ID NO: 4 
AIEKYLKDQAQLNSWGCAFRQVCHTTVPWVNDSLSPDWKNMTWQEWEKQVRYLE
ANISQQLEQAQIQQEKNMYELQKLNSWDVFSNWFDLTSWIKYIQYGVY
SEQ ID NO: 5 
TLTVQARQLLSGIVQQQSNLLRAIEAQQHLLQLTVWGIKQLQTRVLAIERYLQDQQLL
GIWGCSGKLICTTAVPWNSSWSNKSQDEIWDNMTWMQWDKEISNYTYEIYTLIEESQNQ
QEKNEKDLLALDSWKNLWNWFDIT
SEQ ID NO: 6 
AIEKYLKDQAQLNSWGCAFRQVCHTTVPWVNDSLSPDWKNMTWQEWEKQVRYLE
ANISQQLEQAQIQQEKNMYELQKLNSWDVFSNWFDLTSWIKYIQYGVYRSTLTVQARQLL
SGIVQQQSNLLRAIEAQQHLLQLTVWGIKQLQTRVLAIERYLQDQQLLGIWGCSGKLICTT
AVPWNSSWSNKSQDEIWDNMTWMQWDKEISNYTYEIYTLIEESQNQQEKNEKDLLALD
SWKNLWNWFDIT
SEQ ID NO: 7

AIEKYLKDQAQLNSWGSAFRQVSHTTVPWVNDSLSPDWKNMTWQEWEKQVRYLE
ANISQQLEQAQIQQEKNMYELQKLNSWDVFSNWFDLTSWIKYIQYGVYRSTLTVQARQLL
SGIVQQQSNLLRAIEAQQHLLQLTVWGIKQLQTRVLAIERYLQDQQLLGIWGSSGKLISTT
AVPWNSSWSNKSQDEIWDNMTWMQWDKEISNYTYEIYTLIEESQNQQEKNEKDLLALD
SWKNLWNWFDIT

In the specification, phrases such as "one embodiment", "some embodiments", "an example", "specific example", "some examples" indicate that a particular feature, structures, materials, or characteristics described in connection with the embodiment or example isincluded in at least one embodiment or example of the present invention. These phrases do not necessarily refer to the same embodiment or example. Furthermore, the specific features, structures, materials, or characteristics may be combined in any suitable manner in one or more embodiments or examples. In addition, in the absence of conflict, those skilled in the art may combine and integrate different embodiments or examples and the features described herein.

Although the embodiments of the present invention have been shown and described , it can be understood that the above-mentioned embodiments are exemplary and should not be construed as limiting the present invention. Those skilled in the art can make changes, modifications, substitutions, and variations to the above-mentioned embodiments within the scope of the present invention.

### Industrial Applicability

The inventors analyzed gp36 proteins of different HIV-2 strains and designed the gp36 protein sequence as shown in SEQ ID NO: 1. The HIV recombinant antigen designed based on this sequence could effectively detect clinical HIV-2 positive samples with high sensitivity and specificity. When this recombinant HIV antigen protein was used in a label end, it could significantly improve the marker activity of the recombinant HIV antigen, reduce non-specific immune binding activity, and enhance sensitivity.

## Claims

1. An HIV recombinant antigen, wherein the HIV recombinant antigen comprises a segment of amino acids from position x to position y of an HIV-2 gp36 protein, x is any integer from 1 to 60, y is any integer from 143 to 161, and the amino acid sequence of the HIV-2 gp36 protein is as shown in SEQ ID NO: 1.

2. The recombinant antigen according to claim 1, wherein
x is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60; and
y is 143, 144, 145, 146, 147, 148, 149, 150, 151, 152, 153, 154, 155, 156, 157, 158, 159, 160, or 161.

3. The recombinant antigen according to claim 1, wherein the HIV recombinant antigen comprises an HIV-2 gp36 protein sequence as shown in SEQ ID NO: 2, the HIV recombinant antigen comprises an HIV-2 gp36 protein sequence as shown in SEQ ID NO: 3, or the HIV recombinant antigen comprises an HIV-2 gp36 protein sequence as shown in SEQ ID NO: 4.

4. The recombinant antigen according to claim 1, wherein the cysteine in the sequence of the recombinant antigen is mutated to other amino acids; optionally, the cysteine is mutated to serine.

5. The recombinant antigen according to claim 1, wherein the recombinant antigen further comprises segments of other HIV proteins; optionally, the recombinant antigen further comprises a segment of HIV-1; optionally, the recombinant antigen further comprises a segment of HIV-1 gp41; optionally, the recombinant antigen comprises an HIV-1 gp41 protein sequence as shown in SEQ ID NO: 5; and optionally, the recombinant antigen further comprises a fusion partner.

6. The recombinant antigen according to any one of claims 1-5, wherein the recombinant antigen comprises a sequence as shown in SEQ ID NO: 6 or SEQ ID NO: 7.

7. An HIV recombinant antigen, wherein the HIV recombinant antigen comprises any one of the following polypeptides:
a) a polypeptide having the amino acid sequence as shown in SEQ **ID** NO: 1, or a polypeptide derived from the amino acid sequence as shown in SEQ ID NO: 1 by substitution, repetition, deletion, or addition of one or more amino acids and having all or part of the polypeptide activity of the amino acid sequence as shown in SEQ ID NO: 1;
b) a polypeptide having the amino acid sequence as shown in SEQ **ID** NO: 2, or a polypeptide derived from the amino acid sequence as shown in SEQ ID NO: 2 by substitution, repetition, deletion, or addition of one or more amino acids and having all or part of the polypeptide activity of the amino acid sequence as shown in SEQ ID NO: 2;
c) a polypeptide having the amino acid sequence as shown in SEQ ID NO: 3, or a polypeptide derived from the amino acid sequence as shown in SEQ ID NO: 3 by substitution, repetition, deletion, or addition of one or more amino acids and having all or part of the polypeptide activity of the amino acid sequence as shown in SEQ ID NO: 3;
d) a polypeptide having the amino acid sequence as shown in SEQ **ID** NO: 4, or a polypeptide derived from the amino acid sequence as shown in SEQ ID NO: 4 by substitution, repetition, deletion, or addition of one or more amino acids and having all or part of the polypeptide activity of the amino acid sequence as shown in SEQ ID NO: 4;
e) a polypeptide having the amino acid sequence as shown in SEQ **ID** NO: 5, or a polypeptide derived from the amino acid sequence as shown in SEQ ID NO: 5 by substitution, repetition, deletion, or addition of one or more amino acids and having all or part of the polypeptide activity of the amino acid sequence as shown in SEQ ID NO: 5;
f) a polypeptide having the amino acid sequence as shown in SEQ ID NO: 6, or a polypeptide derived from the amino acid sequence as shown in SEQ ID NO: 6 by substitution, repetition, deletion, or addition of one or more amino acids and having all or part of the polypeptide activity of the amino acid sequence as shown in SEQ ID NO: 6; and
g) a polypeptide having the amino acid sequence as shown in SEQ **ID** NO: 7, or a polypeptide derived from the amino acid sequence as shown in SEQ ID NO: 7 by substitution, repetition, deletion, or addition of one or more amino acids and having all or part of the polypeptide activity of the amino acid sequence as shown in SEQ ID NO: 7.

8. A reagent for detecting HIV, wherein the reagent comprises the recombinant antigen according to any one of claims 1-7.

9. The reagent according to claim 8, wherein the recombinant antigen is conjugated with a tracer marker or a solid-phase carrier; the tracer marker is selected from at least one of acridinium ester, luminol, isoluminol, alkaline phosphatase, horseradish peroxidase, colloidal gold, fluorescent microspheres, tris(bipyridine)ruthenium, quantum dot luminescent materials, and up-conversion luminescent materials; and the solid-phase carrier is selected from at least one of magnetic microspheres, plastic microspheres, plastic particles, microplates, glass, capillaries, nylon, and nitrocellulose membranes.

10. A kit for detecting HIV, comprising the recombinant antigen according to any one of claims 1-7, or the reagent according to any one of claims 8-9.

11. A nucleic acid molecule encoding the HIV recombinant antigen according to any one of claims 1-7, a vector comprising the nucleic acid molecule, and a host cell comprising the vector.

12. Use of the recombinant antigen according to any one of claims 1-7 or the reagent according to any one of claims 8-9 in preparation of a kit for detecting HIV.

13. A method for diagnosing an HIV-related disease, comprising: contacting the HIV recombinant antigen according to any one of claims 1-7, the reagent according to any one of claims 8-9, or the kit according to claim 10 with an HIV antibody in a biological sample, and detecting the presence of the HIV-1 antibody in the biological sample; and
optionally, the method comprises performing an immunoassay on the biological sample, wherein the immunoassay comprises ELISA, fluorescent immunochromatography, colloidal gold immunochromatography, chemiluminescence assay, electrochemiluminescence assay, indirect immunofluorescence assay (IFA), or radioimmunoassay (RIA).
